# EUROPÄISCHE PATENTSCHRIFT

(11) **EP 4 003 197 B1**
(45) Veröffentlichungstag und Bekanntmachung des Hinweises auf die Patenterteilung: **19.02.2025**
(21) Anmeldenummer: 20789039.3
(22) Anmeldetag: 05.10.2020
(51) Int. Cl.: A61B 17/70

(54) **IMPLANTAT, INSBESONDERE WIRBELSÄULENIMPLANTAT**
IMPLANT, IN PARTICULAR A SPINAL IMPLANT
IMPLANT, NOTAMMENT IMPLANT VERTÉBRAL

(30) Priorität: 07.10.2019 DE 102019006963
(43) Veröffentlichungstag der Anmeldung: 01.06.2022
(73) Patentinhaber: Signus Medizintechnik GmbH, 63755 Alzenau (DE)
(72) Erfinder: SIEDLER, Uwe, 63755 Alzenau (DE)
(74) Vertreter: Leinweber & Zimmermann
(86) Internationale Anmeldenummer: PCT/EP2020/077874
(87) Internationale Veröffentlichungsnummer: WO 2021/069385

(56) Entgegenhaltungen:
- EP-A1- 2 286 748
- EP-A1- 3 158 957
- US-A1- 2010 145 394
- US-A1- 2015 230 829
- US-A1- 2017 086 895
- US-A1- 2018 014 862
- US-A1- 2019 133 658

## Beschreibung

Die Erfindung betrifft ein Implantat, insbesondere Wirbelsäulenimplantat, in Form einer der Kopplung eines Befestigungselements, insbesondere einer Pedikelschraube, mit einem Verbindungselement, insbesondere einer Verbindungsstange einer Wirbelsäulenstütze dienenden mehrteiligen Verbindungseinrichtung mit einem ersten Teil in Form eines eine Aufnahme für einen Teil des Befestigungselements aufweisenden Sattels und einem zweiten Teil in Form einer in einem axial oberen Bereich eine Aufnahme für das Verbindungselement aufweisenden und in einem axial unteren Bereich die Aufnahme des Sattels umgebenden Tulpe, wobei in einem Montagezustand des in der Tulpe bereits in einer vorgegebenen Orientierung montierten Sattels dieser axial in der Tulpe verschieblich ist, um bei einer Verschiebung in Richtung auf den oberen Bereich in einer Aufnahmeposition den Teil des Befestigungselements aufnehmen zu können und bei einer Verschiebung in Richtung auf den unteren Bereich in einer Halteposition den aufgenommenen Teil des Befestigungselements nicht mehr freigeben zu können, wobei eine Axialführung der axialen Verschieblichkeit einen in einen Ausnehmungsbereich an der radial gesehen Innenseite der Tulpe eingreifenden Vorsprungsbereich an der radial gesehen Außenseite des Sattels aufweist, dessen azimutal gesehen Endbereiche wenigstens 30° voneinander beabstandet sind sowie eine ein axiales Austreten des Vorsprungsbereichs aus dem Ausnehmungsbereich in Richtung auf den oberen Bereich sperrende Axialsperre aufweist.

Derartige Implantate sind bekannt, insbesondere als Wirbelsäulenimplantat für ein Wirbelsäulen-Stützsystem, und z.B. in US 9,486,246 B2 beschrieben. Vereinfacht gesagt geht es darum, zwei oder mehrere Wirbel über eine Wirbelsäulenstütze zu verbinden. Dazu wird in bekannter Weise eine Pedikelschraube in einen Wirbel eingeschraubt und über ein Implantat der eingangs genannten Art mit z.B. einer Verbindungsstange in starrer Kopplung verbunden, wobei andere Wirbel wiederum über Pedikelschrauben über gleiche oder ähnliche Implantate in Form von Verbindungseinrichtungen an diese Verbindungsstange angekoppelt werden. Diese starre Kopplung besteht ausgehend von der oben erwähnten Halteposition, in welcher zudem unter Ausübung einer den Sattel gegen die hülsenartige Tulpe drängenden Axialkraft, die mittels einer in die Tulpe einschraubbaren Feststellschraube erreicht wird und beim Einschrauben direkt auf den Sattel oder mittelbar über die Verbindungsstange auf den Sattel wirkt, wobei durch z.B. eine konische Gestaltung sowohl des unteren Bereichs der Tulpe als auch des Aufnahmebereichs des Sattels eine Klemmkraft auf den Pedikelschraubenkopf und dadurch starre Kopplung zwischen Pedikelschraube und Verbindungsstange erreicht wird.

Um die Pedikelschraube in die Verbindungseinrichtung einzuführen, ist der Sattel aus der Halteposition in eine Aufnahmeposition axial verschieblich. Dort steht dem Aufnahmebereich des Sattels mehr radialer Raum zur Verfügung, um sich aufzuweiten, und in dieser Position lässt sich der Kopf der Pedikelschraube von der Aufnahme des Sattels beispielsweise im Wege eines Aufklipsens umgreifen. In der Aufnahmeposition ist die Orientierung der Axialausrichtung der Pedikelschraube gegenüber derjenigen der Verbindungseinrichtung noch in gewissen Grenzen frei einstellbar (polyaxiale Verbindung), und dies auch in der Halteposition, solange noch keine zu starken Axialkräfte wirken. Aufgrund dieser Gestaltung lässt sich die Verbindungseinrichtung günstig einsetzen, der Operateur kann vor endgültiger Fixierung unter den gegebenen Bedingungen die richtige Positionierung aller Bestandteile der Wirbelsäulenstütze erreichen.

Als Axialführung ist auch bei der oben genannten US 9,486,246 B2 ein Vorsprung am Sattel vorgesehen, der in eine Ausnehmung in der Tulpe eingreift. Der Vorsprung ist in Form einer Drehnase gebildet, um den Sattel passend mit der Tulpe für den Einsatzzustand zu montieren. In diesem sind Einschnitte zwischen Seitenwänden der Tulpe, oftmals U-förmig, und ein Auflagebereich des Sattels für die Verbindungsstange, ebenfalls U-förmig, in dem Montagezustand passend zueinander orientiert, so dass die Verbindungsstange einerseits durch die U-förmige Öffnung der Tulpe gelangt wie auch andererseits auf der U-förmigen Auflage des Sattels zu liegen kommt. Da im Einsatz die Pedikelschraube z.B. auch durch eine untere Öffnung der Tulpe eingeführt und vom Sattel aufgenommen wird, ist die Axialsperre vorgesehen, die ein axiales Austreten des Sattels aus der Tulpe nach oben zuverlässig verhindert, bei dem oben genannten Stand der Technik realisiert durch eine die Ausnehmung begrenzende Schulter, gegen die der Vorsprung/die Drehnase des Sattels anschlägt. Um eine derartige Verbindungseinrichtung einsatzbereit zu machen, wird die Drehnase ausgehend von einer gegenüber dem Einsatzzustand um 90° verdrehten Orientierung des Sattels gegenüber der Tulpe in diese axial eingeführt und dann aus dieser verdrehten Orientierung in die Ausnehmung der Tulpe eingedreht.

US 2010/0145394 A1 lehrt eine innerhalb einer Ebene beschränkte Verschwenkbarkeit einer Pedikelschraube in einer Tulpe. EP 2 286 748 A1 lehrt eine einfache Montage eines Implantats, gemäß der zunächst die Pedikelschraube und anschließend der Sattel von oben in die Tulpe des Implantats eingebracht werden.

US 2018/0014862 A1 offenbart eine Variante, bei welcher der Sattel von unten in die Tulpe eingeführt wird. Auch in US 2017/0086895 A1 ist die Bodenöffnung der Tulpe für eine Axialeinführung des Sattels von unten her ausgelegt. Bei der in US 2015/0230829 A1 offenbarten Variante greifen in Flucht mit dem U-Kanal zur Stabeinführung liegende Vorsprünge des Sattels in in Flucht mit dem U-förmigen Kanal zur Stabeinführung liegende Ausnehmungen der Tulpe ein.

EP 3 158 957 A1 offenbart ein Implantat, welches gemäß der Montage von Sattel und Tulpe ähnlich gestaltet ist wie die oben genannte US 9,486,246 B2.

Der Erfindung liegt die Aufgabe zugrunde, ein Implantat in Form einer Verbindungseinrichtung der eingangs genannten Art insbesondere mit Blick auf seine Zuverlässigkeit zu verbessern.

Diese Aufgabe wird von der Erfindung durch ein Implantat mit den Merkmalen von Anspruch 1 gelöst.

Vorteilhafte Weiterbildungen der Erfindung sind Gegenstand der abhängigen Ansprüche.

Das Implantat weist auf eine gegen ein rotatorisches Eintreten des Vorsprungsbereichs in den Ausnehmungsbereich und im Montagezustand gegen ein rotatorisches Austreten des Vorsprungsbereichs aus dem Ausnehmungsbereich sperrende Drehsperre.

Somit wendet sich die Erfindung von der Lehre der eingangs genannten US 9,486,246 B2 ab, indem das für die dortige Drehnase vorgesehene Ein-(Aus-)Drehen gerade verhindert wird. Im Rahmen der Erfindung ist herausgefunden worden, dass bei erfindungsgemäßer Ausgestaltung eine erhöhte Positionsstabilität der Orientierung von Tulpe und Sattel erreicht wird, und infolgedessen eine erhöhte Lagebeständigkeit der gesamten darauf zurückgreifenden Wirbelsäulenstütze, die sich insbesondere dann positiv auswirkt, wenn die Wirbelsäulenstütze über längere Jahre getragen wird und dadurch gegebenenfalls ein für den Träger unangenehmes frühzeitiges Nachjustieren vermeidet.

Die eingangs genannten Erläuterungen zur polyaxialen Kopplung, Aufnahmeposition und Halteposition gelten auch für die vorliegende Erfindung. Das heißt, im Gebrauch, d.h. beim Implantieren, kann die Verbindungseinrichtung im Montagezustand auf den Kopf einer Polyaxialschraube aufgesetzt werden, wobei in bevorzugten Varianten ein Aufnahmebereich des Sattels auf den Schraubenkopf aufgeklipst wird (in der Aufnahmeposition). Drängt man dann Schraube und Tulpe in unterschiedliche Richtungen (Halteposition), schlägt der untere Bereich des Sattels an den unteren Bereich der hülsenartigen Tulpe, ein Entweichen der Pedikelschraube axial aus der unteren Öffnung der Tulpe ist nicht mehr möglich, wohl aber noch ein Drehen des Kopfes in der Aufnahme gleichbedeutend einem Verschwenken der Tulpe gegenüber der Pedikelschraube (polyaxiale Einstellmöglichkeit). Erst wenn beispielsweise über eine Feststellschraube die in Tulpe/Sattel eingelegte Verbindungsstange fest angekoppelt wird, sorgt die Klemmkraft des Zusammenwirkens von Tulpe und Sattel für ein festes Einklemmen des Schraubenkopfes und somit für eine starre Kopplung zwischen Pedikelschraube und Verbindungsstange der Wirbelsäulenstütze.

Der Vorsprungsbereich wird sich bevorzugt von einem wandartigen Sattelbereich erheben, der selbst flächig und gekrümmt ist, insbesondere im Querschnitt zur Axialrichtung gesehen kreisförmig ist. In einer besonders bevorzugten Ausgestaltung ist die Bogenlänge zwischen den azimutalen Endbereichen des Vorsprungsbereichs um ein Vielfaches, insbesondere wenigstens zweifach, bevorzugt wenigstens dreifach, insbesondere wenigstens vierfach länger als eine Abmessung des Vorsprungsbereichs in der Axialrichtung.

In einer besonders bevorzugten Ausgestaltung sind zwei derartige, insbesondere um 180° versetzt angeordnete Axialführungen vorgesehen, insbesondere in symmetrischer Anordnung. Beide Axialführungen zusammen mit ihren Endbereichen sorgen dafür, dass der Sattel in der Tulpe wenigstens in Bereichen geführt ist, die im Querschnitt gesehen wie die Endpunkte eines "X" verteilt liegen, dessen Mitte durch die Axialachse geht und dessen Oben-Unten-Richtung gegenüber der Lage der Verbindungsstange um 90° verdreht ist.

Erfindungsgemäß weist die Tulpe im oberen Bereich zwei voneinander azimutal beabstandete Seitenwände auf, zwischen denen die Aufnahme für das Verbindungselement liegt. In den Seitenwänden ist jeweils ein Ausnehmungsbereich der Axialführung angeordnet. Diese Seitenwände sind bevorzugt ebenfalls im Querschnitt gesehen bogenförmig, insbesondere kreisförmig.

Bevorzugt ist an der Innenseite der Seitenwände ein Gewinde zum Einschrauben einer Feststellschraube vorgesehen, mittels der insbesondere über das Verbindungselement eine Axialkraft auf den Sattel ausübbar ist. Grundsätzlich sind jedoch auch Ausgestaltungen mit Außengewinde an den Seitenwänden der Tulpe denkbar, und das Heranziehen einer Schraubkappe.

Erfindungsgemäß -weist der Sattel an der seiner Aufnahme abgewandten Seite zwei in der vorgegebenen Orientierung den Seitenwänden entsprechend angeordnete, jeweils einen Vorsprungsbereich einer jeweiligen Axialführung tragende Wände auf. Auch zwischen diesen Wänden ist ein im Axialschnitt bevorzugt U-förmiger Freiraum gebildet, der mit einem entsprechenden Freiraum der Tulpe in Übereinstimmung gebracht einen Aufnahmekanal für die Verbindungsstange bilden kann.

Zur Herstellung des Montagezustands wird der Sattel von oben, d.h. vom axial oberen Bereich her (also der von der Aufnahme für das Teil des Befestigungselements wegweisenden Seite her), in der Axialrichtung der Axialführung in die Tulpe eingeführt (axiales Eintreten von oben).

Erfindungsgemäß weisen die Wände des Sattels eine bei Herstellung des Montagezustands ein axiales Eintreten des Sattels in die Tulpe unter Überwindung der Axialsperre erlaubende elastische Nachgiebigkeit gegen ein radial nach innen gerichtetes Verbiegen auf. Dies erleichtert eine axiale Montage ohne überwiegende plastische Verformung der Bauteile.

In einer zweckmäßigen Ausführung weist das Gewinde an einer oder beiden Seitenwänden eine ein axiales Eintreten des Sattels in die Tulpe erleichternde Einkerbung auf. Dies erleichtert das axiale Einführen bei beibehaltener kompakter Bauweise hinsichtlich der Radialabmessung.

Weitergehend wird es bevorzugt, dass die azimutalen Endbereiche von einem Vorsprungsbereich oder beiden Vorsprungsbereichen wenigstens 36°, insbesondere wenigstens 42° voneinander beabstandet sind. Dies erhöht weiter die Führungsstabilität der Axialführung.

In einer zweckmäßigen Ausführung weist/weisen der/die Ausnehmungsbereiche im Schnitt orthogonal zur Axialrichtung gesehen punktuell oder gesamtheitlich eine zur Form des Vorsprungsbereichs komplementäre Form auf. Dies vermeidet Verkantungen bei der axialen Verschieblichkeit.

In einer weiteren zweckmäßigen Ausführung ist/sind einer oder beide Vorsprungsbereich(e) im Schnitt orthogonal zur Axialrichtung gesehen durchgängig und insbesondere als ein sichelmondförmiger Vorsprung gebildet. Dies erleichtert die Präzisionsgenauigkeit bei der Herstellung des Sattels.

Des Weiteren ist zweckmäßig vorgesehen, dass einer oder beide Vorsprungsbereich(e) im Axialschnitt gesehen nach oben hin eine insbesondere im Wesentlichen rechteckige und/oder nach unten hin eine überstumpfe Stufe bildet/bilden. Dies sorgt für geringe Querkräfte bei Aufrechterhaltung der beabsichtigten Axialsperre und dennoch einer einfacheren Überwindbarkeit der Axialsperre bei Herstellung des Montagezustands.

In einer zweckmäßigen Ausführungsform weisen die Wände des Sattels an ihrer Innenseite eine der Abkopplung eines länglichen Instruments dienende Haltelagerung, insbesondere ein Gewinde auf, welches Instrument zur Freigabe des Befestigungselements durch axiales Halten des Sattels in der Aufnahmeposition bei in dessen Aufnahme aufgenommenem Teil des Befestigungselements dient.

Hinsichtlich der Aufnahme des Sattels ist bevorzugt vorgesehen, dass diese eine den Kopf einer Pedikelschraube insbesondere im Wege des elastischen Aufklipsens haltende und insbesondere eine über einen Bereich des maximalen Kopfdurchmessers hinaus umgreifende Aufnahme ist. Dies sorgt für eine vereinfachte Handhabbarkeit bei noch nicht starrer Kopplung zwischen Schraube und Verbindungseinrichtung.

Ein weiterer Bestandteil der Verbindungseinrichtung kann eine insbesondere einstückige, mit einem passend zum Gewinde der Seitenwände gestalteten Gewinde aufweisende Feststellschraube sein.

Des Weiteren stellt die Erfindung auch einen Implantat-Satz unter Schutz, mit wenigstens einer insbesondere im Querschnitt runden Verbindungsstange, wenigstens zwei Pedikelschrauben und wenigstens zwei Implantaten nach einem der vorgenannten Aspekte im Montagezustand, in welche die Köpfe der Pedikelschrauben durch eine Öffnung am Ende des unteren Bereichs der Tulpe in diese einführbar und von der Aufnahme des Sattels aufnehmbar sind.

Ein solcher Implantat-Satz kann auch noch ein mit einem ein Gewinde passend zum Gewinde der Wände des Sattels gestalteten Gewinde aufweisendes Instrument zum Halten des Sattels gegenüber der Tulpe in der Aufnahmeposition beinhalten.

Weitere Merkmale, Einzelheiten und Vorteile der Erfindung ergeben sich aus der nachfolgenden Beschreibung der Erfindung mit Bezug auf die beiliegenden Figuren, von denen
- Fig. 1: in bereichsweise unterschiedlicher Darstellung einen Sattel in einer Tulpe zeigt,
- Fig. 2: eine Schnittansicht eines unteren Bereichs der Anordnung von Fig. 1 mit gehaltener Pedikelschraube zeigt,
- Fig. 3: eine Sicht auf die Form von Vorsprungsbereichen des Sattels in einer Azimutalebene ist,
- Fig. 4: eine Einkerbung in einem Gewindeabschnitt zeigt,
- Fig. 5: Zwischenstufen einer axialen Montage in perspektivischer Ansicht, Vorderansicht und im Axialschnitt zeigt, und
- Fig. 6: eine andere Gestaltung von Vorsprungsbereichen als die in Fig. 3 gezeigte zeigt.

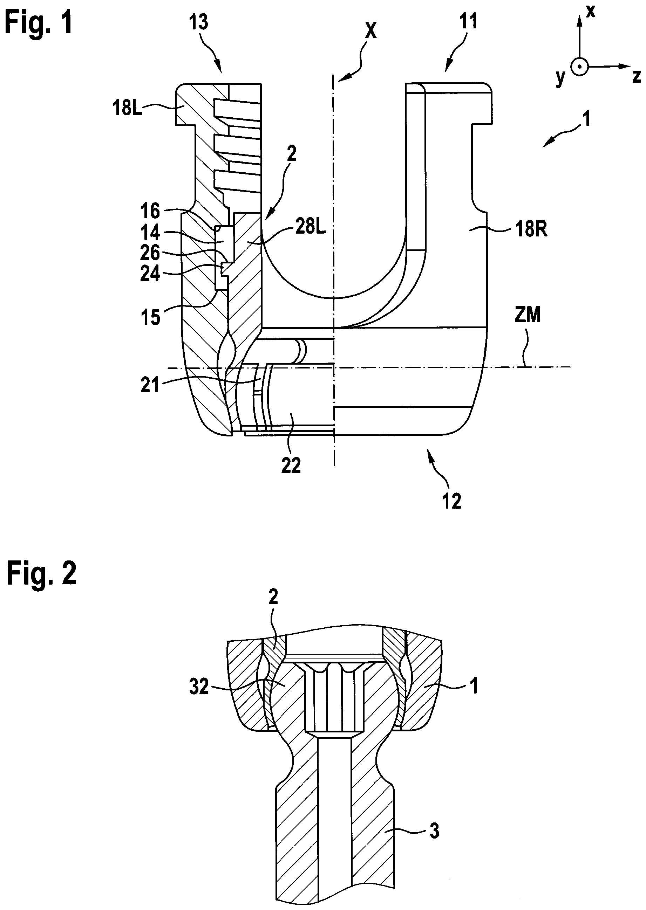

Fig. 1 skizziert den Grundaufbau eines Teils einer zu implantierenden Verbindungseinrichtung, nämlich eine Kombination aus einer Tulpe 1 und einem in der Tulpe 1 angeordneten Sattel 2. In der Darstellung von Fig. 1 zeigt die rechte Bildhälfte eine Vorderansicht, die linke Bildhälfte ist nochmals unterteilt in eine Aufrissansicht im rechten Bereich links der Axialachse X der Verbindungsvorrichtung, und eine Schnittansicht ganz links im Bildbereich.

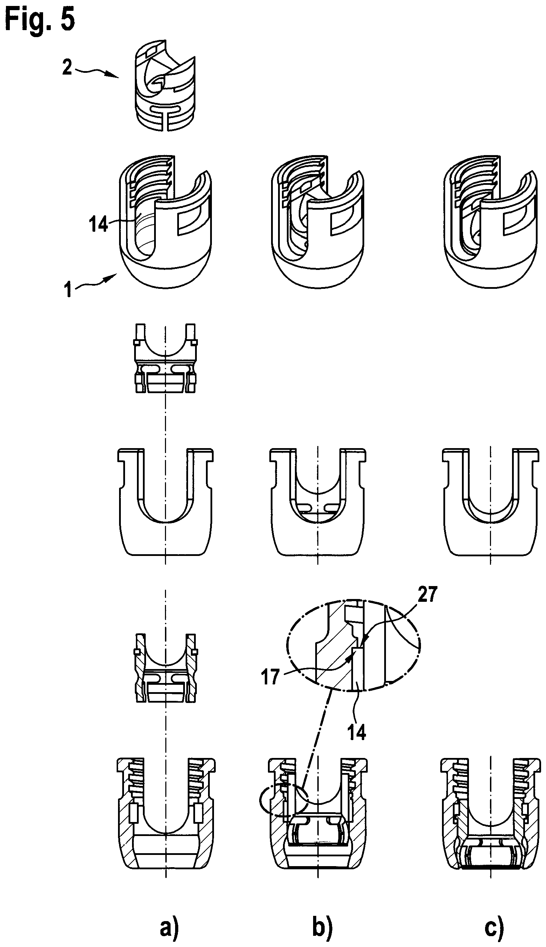

Bezüglich der Axialachse X bildet die Tulpe 1 ein hülsenförmiges Teil mit einem oberen Ende 11 und einem unteren Ende 12. Ausgehend vom oberen Ende 11 ist durch eine U-förmige Ausnehmung in dem dem Betrachter zugewandten und von ihm weggewandten Teil der Hülsenwand ein Kanal mit Verlaufsrichtung Y senkrecht zur Papierebene in Fig. 1 gebildet. Wie aus Fig. 1 und Fig. 5a unmittelbar hervorgeht, weisen die (quer zur Verlaufsrichtung Y des Kanals gesehen) seitlich beidseits der U-förmigen Ausnehmung angeordneten Seitenwände 18L, 18R den Ausnehmungsbereich 14 der Axialführung auf. Durch diesen Kanal lässt sich eine in Fig. 1 nicht gezeigte Verbindungsstange legen, welche in einer dem Fachmann bekannten Art mehrere Verbindungseinrichtungen der gezeigten Art verbindet, welche wiederum mit ebenfalls in Fig. 1 nicht dargestellten Pedikelschrauben gekoppelt sind. Die Verbindungsstange kann dabei lateral (in Y-Richtung) oder auch von oben entlang der Axialrichtung X eingeführt werden, eine Fixierung der Stange mit der Verbindungseinrichtung erfolgt über eine nicht dargestellte Spannschraube, etwa eine Madenschraube, die in das im oberen Bereich der Tulpe 1 gebildete Innengewinde 13 eingeschraubt wird.

Der Sattel 2 weist in seinem oberen Bereich ebenfalls eine U-förmige Ausnehmung auf, die einen Kanal bildet, der in der in Fig. 1 gezeigten, für einen Einsatz vorgesehenen Position parallel zum Kanal der Tulpe 1 verläuft.

In der in Fig. 1 gezeigten, für den Einsatz bereiten Montagestellung ist der Sattel 2 mit einer Axialführung in der Tulpe 1 axial verschieblich. Hierzu ist in den durch den U-förmigen Ausschnitt der Tulpe 1 in ihrem oberen Bereich verbliebenen Seitenwänden 18L, 18R ca. auf halber Höhe der Tulpe 1 eine Ausnehmung 14 gebildet, die in Axialrichtung nach unten durch eine Schulter 15 und in Axialrichtung nach oben durch eine Schulter 16 begrenzt ist. In der Ausnehmung 14 wird ein Vorsprung 24 geführt, der im oberen Bereich des Sattels 2 an der radial gesehen Außenseite der oberen Sattelwand 28L angeordnet ist. Man erkennt, dass der Sattel 2 aus der in Fig. 1 dargestellten Relativstellung gegenüber der Tulpe 1 relativ zu ihr axial nach oben verschieblich ist, bis das obere Ende 26 des Vorsprungs 24 gegen die Schulter 16 schlägt. Auf diese Weise ist eine Axialsperre vorgesehen, die ein weiteres Verschieben des Sattels 2 in der Tulpe 1 in Richtung auf deren oberes Ende 11 verhindert. Die gleiche Axialführung findet sich auf der rechten Seite an der zweiten Wand 18R.

In seinem unteren Bereich ist der Sattel 2 in Form einer körbchenartigen Aufnahme gebildet, mit voneinander durch Spalte 21 voneinander getrennten Halteabschnitten 22. Die körbchenartige Aufnahme kann (Fig. 2) den Kopf 32 einer Pedikelschraube 3 aufnehmen. Wenn beispielsweise eine Pedikelschraube 3 bereits in einen Wirbel eingeschraubt ist, kann die Tulpe 1 mit darin liegendem Sattel 2 auf den Schraubenkopf 32 aufgestülpt werden. Dabei verschiebt sich zunächst der Sattel 2 in der Tulpe 1 nach oben. Sobald der Sattel 2 mit seiner im unteren Bereich maximalen radialen Abmessung in den Bereich (angedeutet durch die gestrichelte Linie ZM) gelangt, in welchem die Tulpe 1 durch eine Innenwölbung einen größeren Innendurchmesser aufweist als am unteren Ende 12, können sich die Halteabschnitte 22 elastisch nach radial außen verbiegen, den Schraubenkopf 32 klipsartig aufnehmen und mit aufgenommenem Schraubenkopf 32 diesen über einen Bereich maximalen Schraubenkopfdurchmessers hinaus übergreifen.

In dieser dem Fachmann bekannten Lage lässt sich die Orientierung der Schraubenachse gegenüber der Axialachse X ausgehend von einer parallelen Lage in jede Raumrichtung jedenfalls um einen gewissen Winkel verschwenken (polyaxiale Kopplung vor der starren Befestigung zwischen Pedikelschraube 3 und Verbindungseinrichtung).

Gemäß ebenfalls einem aus der Technik bekannten Mechanismus wird die letztlich angestrebte starre Kopplung zwischen der Verbindungseinrichtung aus Tulpe 1 und Sattel 2 und der Pedikelschraube 3 dadurch erreicht, dass die in das Gewinde 13 eingeschraubte Spannschraube über die Verbindungsstange den Sattel 2 in der Tulpe 1 nach unten drückt, bis aufgrund der insbesondere konischen Verengung der Öffnung der Tulpe 1 im unteren Bereich zum unteren Ende 12 hin die Halteabschnitte 22 radial nach innen gedrängt werden und die Schraube immer fester bis hin zur starren Kopplung halten (konisches Spannen), was durch Festschrauben der nicht dargestellten Schraube in das Gewinde 13 erreicht wird. Es versteht sich, dass bei bereits leicht angezogener, jedoch noch nicht festgezogener Schraube eine für den implantierenden Chirurgen günstige Situation entsteht, dass er die Relativlage zwischen Schraube und Tulpe 1 durch gezielten Krafteinsatz noch ändern kann, jedoch keine unbeabsichtigte Änderung der Lage mehr eintritt.

Wie besser in der linken oberen Abbildung von Fig. 5 in Spalte a) zu erkennen ist, ist die Ausnehmung 14 in Azimutalrichtung (Drehrichtung in der Y-Z-Ebene) ebenfalls begrenzt und öffnet nicht hin zum U-förmigen Einschnitt zwischen den Seitenwänden 18L und 18R. Der Vorsprungsbereich 24 des Sattels 2 ist demnach, wie in Fig. 5c und Fig. 1 erkennbar, in der Ausnehmung 14 in der jeweiligen Seitenwand 18L, 18R aufgenommen. Die dadurch gebildete Drehsperre verhindert ein Verdrehen des Sattels 2 in der Tulpe 1 mit Axialachse X als Drehachse aus der in Fig. 1 dargestellten Relativlage. Umgekehrt gesehen ließe sich der Sattel 2 auch aus einer gegenüber der in Fig. 1 dargestellten Lage um 90° um die Axialachse X gedrehten Lage nicht in die in Fig. 1 gezeigte Lage bringen, da die Drehsperre bezüglich der Azimutalrichtung in beiden Richtungen wirkt. Das Einsetzen/Einführen des Sattels 2 in die Tulpe 1 kann somit nicht über die Drehbewegung wie im Stand der Technik erfolgen. Vielmehr erfolgt es von oben entlang der Axialachse X, wie aus Fig. 5a erkennbar ist.

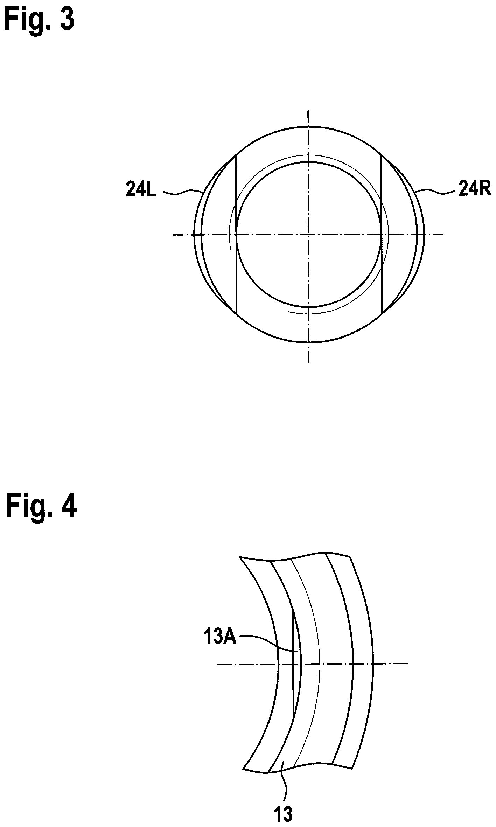

Die Herstellung dieses in Fig. 1 gezeigten Einsatzzustandes, also die Montage der Bauteile Sattel 2 und Tulpe 1 erfolgt rein auf axialem Weg, wie in Fig. 5 dargestellt. Fig. 5 zeigt in jeweils drei Darstellungsarten, von oben nach unten in Perspektivansicht, Vorderansicht und Schnittansicht, Zwischenstufen eines solchen Montagevorgangs. In der linken Darstellung von Fig. 5a) ist dargestellt, wie Sattel 2 oberhalb von Tulpe 1 koaxial zur gemeinsamen Axialachse X mit fluchtenden, durch die jeweiligen U-förmigen Aussparungen gebildeten Kanälen orientiert angeordnet wird. Ausgehend von dieser Lage wird der Sattel 2 axial durch die Öffnung an der oberen Seite 11 der Tulpe 1 in diese eingeführt, zunächst im Wesentlichen kraft- und verformungsfrei, bis der in der mittleren Spalte in Fig. 5b) gezeigte Zustand erreicht ist. Hierzu ist in dem dargestellten Ausführungsbeispiel eine Aussparung 13A in den Gewinderippen des Gewindes 13 vorgesehen. Des Weiteren sind bei diesem Ausführungsbeispiel die Abmessungsverhältnisse in Radialrichtung derart, dass die Vorsprünge 24 beim axialen Einführen den durch die Aussparungen 13A gebildeten Raum auch wenigstens zum Teil nutzen, mit anderen Worten sich das axiale Einführen ohne die Aussparungen 13A erschweren würde. Die Aussparung 13A ist in einem Ausschnitt eines auf Höhe einer Gewinderippe des Gewindes 13 genommenen Querschnitts in Fig. 4 dargestellt.

Ausgehend von der in Fig. 5b) gezeigten Lage ist ein weiteres kraftarmes Einführen aufgrund der vorstehend beschriebenen Axialsperre 16, 26 nicht mehr möglich. Durch dann kraftbeaufschlagtes Einführen werden jedoch die Wände 18L, 18R elastisch radial nach innen gedrängt, bis der Widerstand überwunden und der Vorsprung 24 auf Höhe der Schulter 26 unter elastischer Rückstellung der Wände 18L, 18R in die Ausnehmung 14 gelangen kann. Dann ist die Montagestellung erreicht, die in Fig. 5c) abgebildet ist, welche im Wesentlichen Fig. 1 entspricht.

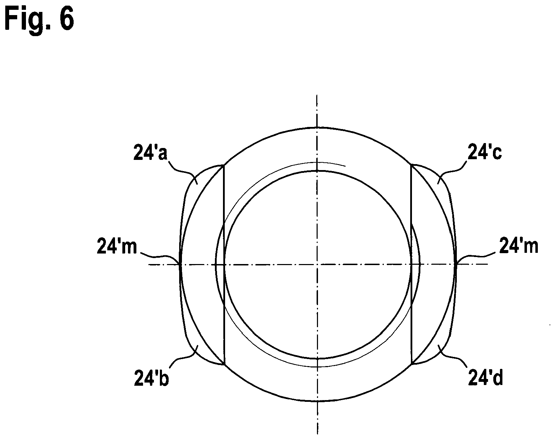

In diesem Ausführungsbeispiel (Fig. 3) ist die azimutale Ausdehnung der Vorsprünge 24L, 24R ca. 50°. Unabhängig von der genauen Form des Vorsprungs 24, insbesondere in einem zentralen Bereich zwischen seinen Endbereichen, wird es bevorzugt, dass die azimutalen Endbereiche des Vorsprungsbereichs einen Azimutalabstand in den oben beschriebenen Bereichen haben, um ein möglichst lagestabiles Führen der Axialführung zu erreichen. Hierzu könnte der Vorsprungsbereich hinsichtlich Materialaufhäufung auch mehr zu den azimutalen Endbereichen verlagert vorgesehen werden, etwa wie in Fig. 6 dargestellt, mehr Gewicht an den Endbereichen 24'a, b, c, d als zentral 24'm.

Wie im Axialschnitt in der vergrößerten unteren Darstellung von Fig. 5b) erkennbar ist, sind die untere Fläche 27 des Vorsprungs 24 und die nach oben weisende Fläche 17 der Schulter 16 nicht orthogonal zur Axialrichtung X gestaltet, sondern konisch, um die Überwindung der Axialsperre bei der Herstellung des Montagezustands zu erleichtern. Im Axialschnitt gesehen kann der Vorsprung 24 somit von der Grundform her eine Stufenform aufweisen, mit einer im Wesentlichen 90°-Stufe für die obere Fläche 26 und einer schrägen Abstufung zur unteren Fläche 27 (mit überstumpfem Winkel).

Im Querschnitt gesehen ist eine sichelmondförmige Gestaltung (Fig. 3) der Vorsprungsbereiche bevorzugt, andere Gestaltungen sind denkbar (etwa Fig. 6), wobei wie oben erläutert die Einhaltung eines Mindestazimutalabstands vorgesehen ist.

## Patentansprüche

1. Implantat in Form einer der Kopplung eines Befestigungselements (3) mit einem Verbindungselement dienenden mehrteiligen Verbindungseinrichtung, mit
einem ersten Teil (2) in Form eines eine Aufnahme für einen Teil des Befestigungselements aufweisenden Sattels (2) und
einem zweiten Teil (1) in Form einer in einem axial oberen Bereich eine Aufnahme für das Verbindungselement aufweisenden und in einem axial unteren Bereich die Aufnahme des Sattels (2) umgebenden Tulpe (1), wobei die Tulpe (1) im oberen Bereich zwei voneinander azimutal beabstandete Seitenwände (18L, 18R) aufweist, zwischen denen die Aufnahme für das Verbindungselement liegt,
wobei in einem Montagezustand des in der Tulpe (1) bereits in einer vorgegebenen Orientierung montierten Sattels (2) dieser axial (X) in der Tulpe (1) verschieblich ist, um bei einer Verschiebung in Richtung auf den oberen Bereich in einer Aufnahmeposition den Teil des Befestigungselements aufnehmen zu können und bei einer Verschiebung in Richtung auf den unteren Bereich in einer Halteposition den aufgenommenen Teil des Befestigungselements nicht mehr freigeben zu können, wobei die Aufnahme des Befestigungselements in polyaxialer Kopplung vorliegt, und das Implantat zur Aufnahme des durch eine untere Öffnung der Tulpe eingeführten Befestigungselements im Sattel ausgelegt ist,
wobei eine Axialführung (14, 24) der axialen Verschieblichkeit einen in einen Ausnehmungsbereich (14) an der radial gesehen Innenseite der Tulpe (1) eingreifenden Vorsprungsbereich (24) an der radial gesehen Außenseite des Sattels (2) aufweist, wobei der Sattel (2) an der seiner Aufnahme abgewandten Seite zwei in der vorgegebenen Orientierung den Seitenwänden (18L, 18R) entsprechend angeordnete, jeweils einen solchen Vorsprungsbereich tragende Wände (28L, 28R) aufweist und wobei der Azimutalabstand der azimutal gesehen Endbereiche des Vorsprungsbereichs (24) wenigstens 30° beträgt, sowie eine ein axiales Austreten des Vorsprungsbereichs (24) aus dem Ausnehmungsbereich (14) in Richtung auf den oberen Bereich sperrende Axialsperre aufweist, und weiter mit
einer gegen ein rotatorisches Eintreten des Vorsprungsbereichs (24) in den Ausnehmungsbereich (14) und im Montagezustand gegen ein rotatorisches Austreten des Vorsprungsbereichs (24) aus dem Ausnehmungsbereich (14) sperrende Drehsperre, und
wobei Tulpe (1) und Sattel (2) für eine Herstellung des Montagezustands durch Einführung des Sattels (2) in die Tulpe (1) in Axialrichtung der Axialführung gesehen axial von oben ausgelegt sind und hierzu die Wände (28L, 28R) des Sattels (2) eine bei Herstellung des Montagezustands ein axiales Eintreten des Sattels (2) in die Tulpe (1) unter Überwindung der Axialsperre erlaubende elastische Nachgiebigkeit gegen ein radial nach innen gerichtetes Verbiegen aufweisen.

2. Implantat nach Anspruch 1, mit zwei derartigen, insbesondere um 180° versetzt angeordneten Axialführungen.

3. Implantat nach Anspruch 1 oder 2, mit einem an der Innenseite der Seitenwände (18L, 18R) vorgesehenen Gewinde (13) zum Einschrauben einer Feststellschraube, mittels der insbesondere über das Verbindungselement eine Axialkraft auf den Sattel (2) ausübbar ist.

4. Implantat nach einem der Ansprüche 1 bis 3, bei dem das Gewinde (13) an einer oder beiden Seitenwänden eine ein axiales Eintreten des Sattels (2) in die Tulpe (1) erleichternde Einkerbung (13A) aufweist.

5. Implantat nach einem der Ansprüche 1 bis 4, bei dem der Azimutalabstand der azimutalen Endbereiche von einem Vorsprungsbereich oder beiden Vorsprungsbereichen wenigstens 36°, insbesondere wenigstens 42° beträgt.

6. Implantat nach einem der vorhergehenden Ansprüche, bei dem der/die Ausnehmungsbereich(e) im Schnitt orthogonal zur Axialrichtung gesehen eine zur Form des Vorsprungsbereichs komplementäre Form aufweist/aufweisen.

7. Implantat nach Anspruch 6, bei dem einer oder beide Vorsprungsbereich(e) im Schnitt orthogonal zur Axialrichtung gesehen durchgängig und insbesondere als ein sichelmondförmiger Vorsprung gebildet ist/sind.

8. Implantat nach Anspruch 7, bei dem einer oder beide Vorsprungsbereich(e) im Axialschnitt gesehen nach oben hin eine insbesondere im Wesentlichen rechteckige und/oder nach unten hin eine überstumpfe Stufe bildet/bilden.

9. Implantat nach einem der vorhergehenden Ansprüche, bei dem die Wände des Sattels (2) an ihrer Innenseite ein der Ankopplung eines länglichen Instruments dienendes Gewinde aufweisen, welches Instrument zur Freigabe des Befestigungselements durch axiales Halten des Sattels (2) in der Aufnahmeposition bei in dessen Aufnahme aufgenommenem Teil des Befestigungselements dient.

10. Implantat nach einem der vorhergehenden Ansprüche, bei dem die Aufnahme des Sattels (2) eine den Kopf einer Pedikelschraube insbesondere im Wege des elastischen Aufklipsens haltende und insbesondere eine über einen Bereich des maximalen Kopfdurchmessers hinaus umgreifende Aufnahme (22) ist.

11. Implantat nach einem der vorhergehenden Ansprüche, mit einer ein passend zum Gewinde (13) der Seitenwände gestaltetes Gewinde aufweisenden Feststellschraube.

12. Wirbelsäulenimplantat, gebildet durch ein Implantat nach einem der vorhergehenden Ansprüche, bei dem die mehrteilige Verbindungseinrichtung der Kopplung einer Pedikelschraube mit einer Verbindungsstange einer Wirbelsäulenstütze dient.

13. Implantat-Satz mit wenigstens einer insbesondere im Querschnitt runden Verbindungsstange, wenigstens zwei Pedikelschrauben und wenigstens zwei Implantaten nach einem der Ansprüche 1 bis 12 im Montagezustand, in welche die Köpfe der Pedikelschrauben durch eine Öffnung am Ende (12) des unteren Bereichs der Tulpe (1) in diese einführbar und von der Aufnahme des Sattels (2) aufnehmbar sind.

14. Implantat-Satz nach Anspruch 13, mit einem ein Gewinde passend zum Gewinde der Wände (28) des Sattels (2) gestalteten Gewinde aufweisenden Instrument zum Halten des Sattels (2) gegenüber der Tulpe (1) in der Aufnahmeposition.

## Claims

1. An implant formed as a multi-part connecting device for coupling a fastening element (3) to a connecting element, comprising:
a first part (2) formed as a saddle (2) having a receptacle for a part of the fastening element; and
a second part (1) formed as a tulip (1) which, in an axially upper region, has a receptacle for the connecting element and, in an axially lower region, surrounds the receptacle of the saddle (2), wherein the tulip (1) has, in the upper region, two azimuthally spaced side walls (18L, 18R), between which the receptacle for the connecting element sits,
wherein, in an assembly state of the saddle (2) already mounted in a predefined orientation in the tulip (1), the saddle is movable axially (X) in the tulip (1) so that, when moved in the direction of the upper region, said saddle in a receiving position is able to receive the part of the fastening element, and so that, when moved in the direction of the lower region, said saddle in a holding position is no longer able to release the received part of the fastening element, wherein the fastening element is received in a polyaxial coupling and the implant is configured for receiving, in the saddle, the fastening element inserted through a lower opening of the tulip,
wherein an axial guide (14, 24) that provides axial mobility comprises a projection region (24) on an outer face of the saddle (2), when viewed radially, which engages a recess region (14) on an inner face of the tulip (1), when viewed radially, wherein the saddle (2), on a face facing away from the receptacle, has two walls (28L, 28R) arranged in the predefined orientation corresponding to the side walls (18L, 18R) of the tulip, each wall bearing such a projection region, and wherein the azimuth spacing of the end regions of the projection region (24), when viewed azimuthally, is at least 30°, and wherein the axial guide also comprises an axial lock that blocks the projection region (24) against axially exiting the recess region (14) in the direction of the upper region; wherein the implant further comprises:
a rotary lock that prevents the projection region (24) from rotationally entering the recess region (14) and, in the assembly state, prevents the projection region (24) from rotationally exiting the recess region (14); and
wherein the tulip (1) and saddle (2) are configured to produce the assembly state by inserting the saddle (2) into the tulip (1) axially from above, when viewed in the axial direction of the axial guide and to this end the walls (28L, 28R) of the saddle (2) have an elastic resilience against radially inwardly directed bending, which allows the saddle (2) to enter the tulip (1) axially, overcoming the axial lock, when the assembly state is produced.

2. The implant according to claim 1, comprising two such axial guides, in particular axial guides arranged with a 180° offset.

3. The implant according to claim 1 or 2, comprising a thread (13), provided on the inner face of the side walls (18L, 18R), for screwing in a locking screw by means of which an axial force can be exerted on the saddle (2), in particular via the connecting element.

4. The implant according to one of claims 1 to 3, wherein the thread (13) has, on one or both side walls, a notch (13A) that facilitates axial entry of the saddle (2) into the tulip (1).

5. The implant according to one of claims 1 to 4, wherein the azimuthal spacing of the azimuthal end regions of one projection region or both projection regions is at least at least 36°, in particular at least 42°.

6. The implant according to one of the preceding claims, wherein the recess region(s), when viewed in a section orthogonal to an axial direction, has/have a shape that complements a shape of the projection region.

7. The implant according to claim 6, wherein one or both projection region(s), when viewed in a section orthogonal to the axial direction, is/are continuous and in particular is/are formed as a crescent-shaped projection.

8. The implant according to claim 7, wherein one or both projection region(s), when viewed in an axial section, forms/form, in an upward direction, an in particular substantially rectangular step and/or, in a downward direction, a reflex step.

9. The implant according to one of the preceding claims, wherein the walls of the saddle (2) have, on their inner face, a thread for coupling an elongate instrument, which instrument serves to release the fastening element by axially holding the saddle (2) in the receiving position while part of the fastening element is received in the receptacle of the saddle.

10. The implant according to one of the preceding claims, wherein the receptacle of the saddle (2) is a receptacle (22) that holds the head of a pedicle screw, in particular by means of elastic clipping, and in particular engages beyond a region of the maximum diameter of the head of the pedicle screw.

11. The implant according to one of the preceding claims, comprising a locking screw having a thread that matches the thread (13) of the side walls.

12. A spinal implant, formed by an implant according to one of the preceding claims, wherein the multi-part connecting device is used to couple a pedicle screw to a connecting rod of a spinal support.

13. An implant set comprising at least one connecting rod, which in particular has a round cross-section, at least two pedicle screws and at least two implants according to one of claims 1 to 12 in the assembly state, wherein the heads of the pedicle screws can be inserted into the tulip (1) through an opening at the end (12) of the lower region of the tulip (1) and can be received by the receptacle of the saddle (2).

14. The implant set according to claim 13, comprising an instrument having a thread matching the walls (28) of the saddle (2), for holding the saddle (2) in the receiving position relative to the tulip (1).

## Revendications

1. Implant se présentant sous la forme d'un dispositif de raccord en plusieurs pièces et servant à coupler un élément de fixation (3) à un élément de raccord, et comprenant :
une première pièce (2) en forme d'étrier (2), présentant un réceptacle pour une partie de l'élément de fixation, et
une deuxième pièce (1) en forme de tulipe (1), présentant un réceptacle pour l'élément de raccord dans une région axialement supérieure et entourant le réceptacle de l'étrier (2) dans une région axialement inférieure, ladite tulipe (1) présentant, dans la région supérieure, deux parois latérales (18L, 18R) espacées azimutalement, entre lesquelles se trouve le réceptacle pour l'élément de raccord ;
dans lequel, dans un état assemblé de l'étrier (2) déjà monté dans la tulipe (1) selon une orientation prédéterminée, celui-ci est déplaçable axialement (X) dans la tulipe (1) vers une position de réception lui permettant de recevoir la partie de l'élément de fixation après un déplacement en direction de la région supérieure et vers une position de retenue lui permettant de ne plus libérer ladite partie de l'élément de fixation reçue après un déplacement en direction de la région inférieure, la réception de l'élément de fixation étant en couplage polyaxial, et l'implant étant conçu pour recevoir, dans l'étrier, l'élément de fixation introduit par une ouverture inférieure de la tulipe,
dans lequel un moyen de guidage axial (14, 24) servant au déplacement axial présente une région en saillie (24) sur l'extérieur - vu radialement - de l'étrier (2) qui coopère avec une région d'évidement (14) présente sur l'intérieur - vu radialement - de la tulipe (1), ledit étrier (2) présentant, du côté opposé à son réceptacle, deux parois (28L, 28R) chacune porteuse d'une région en saillie et agencées, selon l'orientation prédéterminée, en correspondance avec les parois latérales (18L, 18R), ledit espacement azimutal des régions terminales - vu azimutalement - de la région en saillie (24) étant d'au moins 30°, ledit moyen de guidage axial présentant également un blocage axial qui empêche la sortie axiale de la région en saillie (24) hors de la région d'évidement (14) en direction de la région supérieure ; ledit implant comprenant en outre :
un blocage antirotation empêchant une entrée par rotation de la région en saillie (24) dans la région d'évidement (14) et, à l'état assemblé, empêchant une sortie par rotation de la région en saillie (24) hors de la région d'évidement (14),
ladite tulipe (1) et ledit étrier (2) étant conçus pour produire l'état assemblé par introduction de l'étrier (2) dans la tulipe (1) axialement depuis le dessus, vu dans la direction axiale du moyen de guidage axial et, à cet effet, les parois (28L, 28R) de l'étrier (2) présentent une flexibilité élastique contre un gauchissement dirigé radialement vers l'intérieur, ce qui permet à l'étrier (2) d'entrer axialement dans la tulipe (1) tout en surmontant le blocage axial lors de la production de l'état assemblé.

2. Implant selon la revendication 1, comprenant deux desdits moyens de guidage axial, notamment disposés en décalage de 180°.

3. Implant selon la revendication 1 ou 2, comprenant un filetage (13) prévu du côté intérieur des parois latérales (18L, 18R) pour le vissage d'une vis de blocage, au moyen de laquelle une force axiale peut être exercée sur l'étrier (2), notamment par l'intermédiaire de l'élément de raccord.

4. Implant selon l'une des revendications 1 à 3, dans lequel le filetage (13) présente une encoche (13A) sur l'une ou les deux parois latérales pour faciliter l'entrée axiale de l'étrier (2) dans la tulipe (1).

5. Implant selon l'une des revendications 1 à 4, dans lequel l'espacement azimutal des régions terminales azimutales d'une ou des deux régions en saillie est d'au moins 36°, notamment d'au moins 42°.

6. Implant selon l'une des revendications précédentes, dans lequel la ou les régions d'évidement ont une forme complémentaire de la forme de la région en saillie, vu en coupe orthogonalement à la direction axiale.

7. Implant selon la revendication 6, dans lequel l'une ou les deux régions en saillie sont continues, vu en coupe orthogonalement à la direction axiale, et ont notamment la forme d'une saillie en croissant de lune.

8. Implant selon la revendication 7, dans lequel l'une ou les deux régions en saillie, vu en coupe axiale, forment, vers le haut, un gradin notamment sensiblement rectangulaire et/ou, vers le bas, un gradin rentrant.

9. Implant selon l'une des revendications précédentes, dans lequel les parois de l'étrier (2) présentent, sur leur intérieur, un filetage permettant d'accoupler un instrument allongé, lequel instrument sert à libérer l'élément de fixation en maintenant axialement l'étrier (2) en position de réception tandis que la partie de l'élément de fixation est reçue dans son réceptacle.

10. Implant selon l'une des revendications précédentes, dans lequel le réceptacle de l'étrier (2) est un réceptacle (22) qui maintient la tête d'une vis pédiculaire, notamment au moyen d'un clipsage élastique, et qui, notamment, entoure ladite tête au-delà d'une région de diamètre maximal de celle-ci.

11. Implant selon l'une des revendications précédentes, comprenant une vis de blocage présentant un filetage adapté au filetage (13) des parois latérales.

12. Implant rachidien, formé par un implant selon l'une des revendications précédentes, dans lequel le dispositif de raccord en plusieurs pièces sert à coupler une vis pédiculaire à une tige de raccord d'un support rachidien.

13. Ensemble d'implants comprenant au moins une tige de raccord, notamment à section ronde, au moins deux vis pédiculaires et au moins deux implants selon l'une des revendications 1 à 12 à l'état assemblé dans lesquels les têtes des vis pédiculaires peuvent être introduites à travers une ouverture à l'extrémité (12) de la région inférieure de la tulipe (1) et peuvent être reçues dans le réceptacle de l'étrier (2).

14. Ensemble d'implants selon la revendication 13, comprenant un instrument présentant un filetage adapté au filetage des parois (28) de l'étrier (2) et servant à retenir l'étrier (2) par rapport à la tulipe (1) en position de réception.
